Europäisches Patentamt

European Patent Office

Office européen des brevets

(11) Publication number: **0 233 382 B1**

## EUROPEAN PATENT SPECIFICATION

(45) Date of publication of patent specification: **22.01.92** (51) Int. Cl.⁵: **C07C 2/12**, B01J 29/18

(21) Application number: **86202161.5**

(22) Date of filing: **03.12.86**

(54) Process for the preparation of liquid hydrocarbons.

(30) Priority: **23.12.85 GB 8531628**

(43) Date of publication of application:
**26.08.87 Bulletin 87/35**

(45) Publication of the grant of the patent:
**22.01.92 Bulletin 92/04**

(84) Designated Contracting States:
**AT BE CH DE FR GB IT LI NL SE**

(56) References cited:
**GB-A- 1 135 938**
**GB-A- 1 216 272**
**US-A- 3 542 671**
**US-A- 4 423 269**

(73) Proprietor: **SHELL INTERNATIONALE RE-
SEARCH MAATSCHAPPIJ B.V.
Carel van Bylandtlaan 30
NL-2596 HR Den Haag(NL)**

(72) Inventor: **Germaine, Gilbert
Badhuisweg 3
NL-1031 CM Amsterdam(NL)**

(74) Representative: **Spierenburg, Jan et al
Shell Internationale Research Maatschappij
B.V. Patents, Licensing & Trade Marks Di-
vision P.O. Box 302
NL-2501 CH The Hague(NL)**

Rank Xerox (UK) Business Services

EP 0 233 382 B1

EP 0 233 382 B1

**Description**

The invention relates to a process for the preparation of liquid hydrocarbons and to a catalytically active composition suitable for use in said process.

It is known to apply crystalline aluminium silicates (e.g. mordenite), into which one or more catalytically active metals have been incorporated, in a wide variety of hydrocarbon conversion processes such as hydrocracking, isomerization and oligomerization. For example, US 3,542,671 describes a hydroisomerization process for the conversion of olefins to isoparaffins using, for example, an H-faujasite or H-mordenite catalyst containing a Group VI or VIII metal hydrogenation promotor.

It has now surprisingly been found that liquid hydrocarbons can be selectively prepared from an olefinic hydrocarbons-containing feed which olefins are converted to higher molecular weight products in a very stable operation by employing a mordenite-type of catalyst comprising a catalytically active metal in a specific molar ratio.

The invention therefore relates to a process for the preparation of liquid hydrocarbons which comprises contacting an olefinic hydrocarbons-containing feed at elevated temperature and pressure with a catalyst comprising at least one n-valent metal (X) selected from the group consisting of metals from Groups 1b, 2a, 2b, 4b, 5b, 6b, 7b and 8 of the Periodic Table of the Elements on a mordenite-type of trivalent metal (Y) silicate as carrier in which catalyst the molar ratio X : Y is greater than $n^{-1}$.

Reference is being made to the Periodic Table of the Elements as published in the "Handbook of Chemistry and Physics", 55th edition (1975), CRC Press, Ohio, U.S.A.

The invention furthermore relates to a catalytically active composition which comprises at least one n-valent metal (X) selected from the group consisting of metals from Groups 1b, 2a, 2b, 4b, 5b, 6b, 7b and 8 of the Periodic Table of the Elements on a mordenite-type of trivalent metal (Y) silicate as carrier in which composition the molar ratio X : Y is greater than $n^{-1}$.

The catalytically active composition according to the invention will hereinafter be referred to as catalyst employed in the process according to the invention.

Preferably, at least part of the amount, and most preferably the total amount, of metal(s) X has(have) been incorporated into the catalyst by means of ion exchange. Any ion exchange technique known in the art can be suitably applied. Preferably, the catalyst applied in the process according to the invention is prepared by treating the mordenite-type of carrier material, which comprises exchangeable cations such as alkali metal-, hydrogen- and/or preferably ammonium ions, one or more times with a solution of at least one metal (X) salt such as an aqueous solution of a metal (X) nitrate or -acetate. The ion exchange treatment is suitably carried out at a temperature from 0 °C up to the boiling temperature of the solution, and preferably at a temperature from 20 - 100 °C.

The valency n of the metals X can vary from +1 to +6. Preferably, however, at least one of the metals X in the catalyst is bivalent in which case the molar ratio X : Y is greater than 0.5. X is preferably selected from the group consisting of the bivalent metals copper, zinc, cadmium, magnesium, calcium, strontium, barium, titanium, vanadium, chromium, manganese, iron, cobalt and nickel. A particularly preferred element X is nickel.

The trivalent metal Y which is present in the crystal structure of the mordenite-type of metal silicate catalyst carrier preferably comprises at least one metal selected from the group consisting of aluminium, iron, gallium, rhodium, chromium and scandium. Most preferably Y consists substantially of aluminium; the resulting crystalline aluminium silicate preferably comprises a major part of mordenite and most preferably consists substantially completely of mordenite.

The molar ratio silicon : Y in the catalyst is suitably in the range from 5:1 to 100:1 and preferably in the range from 7:1 to 30:1. This ratio is in most cases substantially identical to the molar ratio Si : Y in the crystalline metal silicate employed as carrier material, except when some of the metal Y has been removed from the crystal structure during the catalyst preparation e.g. by means of acid leaching.

If desired (e.g. in order to increase the crushing strength of the catalyst particles), the carrier material and/or the ready catalyst can be combined with a binder material such as (a combination of) refractory oxide(s), clay and/or carbon. Suitable refractory oxides comprise alumina, silica, magnesia, zirconia, titania and combinations thereof.

The molar ratio X : Y in the ready catalyst is preferably from 0.6 - 1.5 and most preferably from 0.8 - 1.2. A molar ratio X : Y of 0.5 or less for bivalent metals X, which is outside the scope of the present invention, results in a catalyst which is considerably less stable than a catalyst for which said ratio is greater than 0.5. A very high molar ratio X : Y of e.g. more than 2 could lead to difficulties in the catalyst preparation and result in a relatively inactive catalyst with a relatively low surface area and pore volume due to the very high degree of loading with metal(s) X.

2

After loading of the carrier material with the metal(s) X, the catalytically active composition thus obtained is preferably dried and calcined before being employed as catalyst in the process according to the present invention. Drying is suitably carried out at a temperature from 100-400 °C, and preferably from 110-300 °C, for a period of 1-24 hours; the calcination temperature is suitably from 400-800 °C and preferably from 450-650 °C. The calcination treatment is suitably carried out at (sub-)atmopheric or elevated pressure for a period of 0.1-24 hours, and preferably of 0.5-5 hours in air or in an inert (e.g. nitrogen) atmosphere.

A wide variety of olefinic hydrocarbons-containing feeds can be employed in the process according to the present invention, provided that the dimensions of the olefinic hydrocarbon molecules are such that they can be catalytically converted with a mordenite-type of catalyst.

Alpha-olefins, and in particular alpha-mono olefins are preferably used as feed (components). However, internal olefins such as butene-2, which may be isomerized at the prevailing process conditions, are also suitably used as feed (components). Preferably, the feed contains more than 50% by weight of olefins having at most six carbon atoms per molecule ($C_6^-$ olefins) such as ethene, propene, n-butenes, isobutene, n-pentenes, isopentenes, n-hexenes and isohexenes; in addition to said olefins, aliphatic hydrocarbons such as (cyclic) paraffins, di-olefins and mono-olefins having more than six carbon atoms per molecule can be present in the feed.

Special preference is given to ethene- and/or propene-containing feeds which are suitably obtained as by-product from (fluid) catalytic cracking processes, thermal cracking processes (e.g. for the preparation of ethene), coking- and/or pyrolysis processes.

Suitable feeds for the present process can also be prepared starting from synthesis gas which is first converted into methanol and subsequently into a product substantially consisting of $C_6^-$ olefins. Alternatively, the synthesis gas can be converted in the presence of a Fischer-Tropsch type of catalyst into a product which in addition to paraffinic hydrocarbons contains a considerable amount of $C_6^-$ olefins.

The process according to the invention is preferably carried out at a temperature from 150-330 °C, a pressure from 1-100 bar abs. and a space velocity from 0.1-10 kg feed/kg catatlyst.hour. Most preferably, the process is carried out at a temperature from 180-300 °C, a pressure from 10-50 bar abs. and a space velocity from 0.2-5 kg feed/kg catalyst.hour.

The process according to the invention can be carried out in one or more fixed-, moving- and/or fluidized beds; preferably, the process is carried out in a fixed bed of catalyst particles such as extrudates, pellets or spheres passing sieve openings having a width from 0.05-5 mm, and preferably from 0.1-1 mm.

Liquid hydrocarbons prepared by a process as described hereinbefore include products boiling in the gasoline range (40-150 °C), the middle distillate range (kerosene- and gasoilfractions boiling from 150-370 °C) and in the lubricating base oil range (above 370 °C). Products boiling below the gasoline boiling range and unconverted feed, if any, are preferably separated off from the normally liquid products and can be recycled, if desired.

The invention is illustrated the following Examples.

EXAMPLE 1: preparation of catalyst A.

Modenite in the ammonium form with a molar ratio Silicon : aluminium (= Y) of 9 is ion exchanged at a temperature of 100 °c with an ads solution containing one mol nickel(II) acetate/-litre. The resulting catalyst has a molar ratio of nickel (= X) : aluminium (= Y) of one, is dried at a temperature of 120 °c for 16 furs and calcined in air at a temperature of 500 °C for one hour to obtain catalyst A.

Comparative example. Comparative catalyst L (having a molar ratio of nickel : aluminium of 0.2) is prepared in a similar manner as catalyst A, except that a solution containing 0.1 mol nickel(II) acetate/litre is used for its preparation.

EXAMPLE 2: preparation of liquid hydrocarbons.

Propene is led with a space velocity of 0.5 kg propene/kg catalyst.hour through a microflow reactor containing 6 g of catalyst with a particle size of 0.2-0.6 mm at a temperature of 215 °C and a pressure of 16 bar abs.

The test results are given in the following Table, in which $C_{24}$ and $C_{400}$ represent the conversion of propene (expressed as a weight percentage Used on propene feed) after a test duration of 24 and 400 hours, respectively; similarly, $S_{24}$ and $S_{400}$ represent the selectivity for products having at least 5 carbon atoms per molecule ($C_{5+}$) defined as the weight percentage of $C_{5+}$ in the total product (liquid and gas).

3

TABLE

| Catalyst | $C_{24}$ | $C_{400}$ | $S_{24}$ | $S_{400}$ |
|----------|----------|-----------|----------|-----------|
| A | 100 | 100 | 100 | 99.8 |
| L | 100 | 79 | 100 | 97.2 |

From the results given in the Table it is clear that catalyst A according to the invention is very stable whereas the conversion decreased substantially for comparative catalyst L after 400 hours of testing.

**Claims**

1. Process for the preparation of liquid hydrocarbons which comprises contacting an olefinic hydrocarbons-containing feed at elevated temperature and pressure with a catalyst comprising at least one n-valent metal (X) selected from the group consisting of metals from Groups 1b, 2a, 2b, 4b, 5b, 6b, 7b and 8 of the Periodic Table of the Elements on a mordenite-type of trivalent metal (Y) silicate as carrier in which catalyst the molar ratio X : Y is greater than $n^{-1}$.

2. Process according to claim 1 wherein the metal(s) X has(have) been incorporated into the catalyst by means of ion exchange.

3. Process according to claim 1 or 2 wherein at least one of the metals X is bivalent.

4. Process according to any one of the preceding claims wherein X comprises nickel.

5. Process according to any one of the preceding claims wherein Y comprises at least one metal selected from the group consisting of aluminium, iron, gallium, rhodium, chromium and scandium.

6. Process according to any one of the preceding claims wherein the molar ratio silicon : Y in the catalyst is in the range from 5:1 to 100:1 and preferably in the range from 7:1 to 30:1.

7. Process according to any one of the preceding claims wherein the molar ratio X : Y is from 0.6-1.5 and preferably from 0.8-1.2.

8. Process according to any one of the preceding claims wherein the catalyst has been prepared from a carrier comprising ammonium mordenite.

9. Process according to any one of the preceding claims wherein the feed comprises alpha-mono olefins.

10. Process according to any one of the preceding claims which is carried out at a temperature from 150-330 °C, a pressure from 1-100 bar abs. and a space velocity from 0.1-10 kg feed/kg catalyst.hour.

11. Catalytically active composition which comprises at least one n-valent metal (X) selected from the group consisting of metals from Groups 1b, 2a, 2b, 4b, 5b, 6b, 7b and 8 of the Periodic Table of the Elements on a mordenite-type of trivalent metal (Y) silicate as carrier in which composition the molar ratio X : Y is greater than $n^{-1}$.

**Revendications**

1. Procédé pour la préparation d'hydrocarbures liquides, qui consiste à mettre en contact une charge renfermant des hydrocarbures oléfiniques à température et sous pressions élevées avec un catalyseur renfermant au moins un métal n-valent (X) choisi dans le groupe constitué par les métaux des groupes 1b, 2a, 2b, 4b, 5b, 6b, 7b et 8 du Tableau périodique des éléments sur un silicate de métal trivalent (Y) de type mordénique, en tant que support, procédé dans lequel le rapport molaire X:Y est supérieur à $n^{-1}$.

2. Procédé selon la revendication 1, dans lequel le métal (les métaux) X a (ont) été incorporé(s) dans le catalyseur par échange d'ions.

**3.** Procédé selon la revendication 1 ou 2, dans lequel au moins l'un des métaux X est bivalent.

**4.** Procédé selon l'une quelconque des revendications précédentes, dans lequel X comprend du nickel

**5.** Procédé selon l'une quelconque des revendications précédentes, dans lequel Y comprend au moins un métal choisi dans le groupe constitué par l'aluminium, le fer, le gallium, le rhodium, le chrome et le scandium.

**6.** Procédé selon l'une quelconque des revendications précédentes, dans lequel le rapport molaire du silicium à Y dans le catalyseur se situe dans la gamme de 5:1 à 100:1 et de préférence dans la gamme de 7:1 à 30:1.

**7.** Procédé selon l'une quelconque des revendications précédentes, dans lequel le rapport molaire de X à Y est de 0,6 à 1,5 et de préférence de 0,8 à 1,2.

**8.** Procédé selon l'une quelconque des revendications précédentes, dans lequel le catalyseur a été préparé à partir d'un support renfermant de la mordénite ammonide.

**9.** Procédé selon l'une quelconque des revendications précédentes, dans lequel la charge comprend des alpha-mono-oléfines.

**10.** Procédé selon l'une quelconque des revendications précédentes, lequel est mis en oeuvre à une température de 150 à 350°C, sous une pression de 1 à 100 bars absolus et une vitesse spatiale de 0,1 à 10 kg de charge par kg de catalyseur et par heure.

**11.** Composition active du point de vue catalytique, qui comprend au moins un métal n-valent (X) choisi dans le groupe constitué des métaux des groupes 1b, 2a, 2b, 4b, 5b, 6b, 7b et 8 du Tableau périodique des éléments sur un silicate de métal trivalent (Y) du tue mordénite, en tant que support, composition dans laquelle le rapport molaire X:Y est supérieur à $n^{-1}$.

**Patentansprüche**

**1.** Verfahren zur Herstellt von flüssigen Kohlenwasserstoffen, welches ein In-Berührung-Bringen eines olefinische Kohlenwasserstoffe enthaltenden Einsatzmaterials bei erhöhter Temperatur und erhöhtem Druck mit einem Katalysator umfaßt, der wenigstens ein n-wertiges Metall (X), ausgewählt aus der aus Metallen der Gruppen 1b, 2a, 2b, 4b, 5b, 6b, 7b und 8 des Periodensystems der Elemente bestehenden Gruppe, auf einem Silikat von dreiwertigem Metall (Y) vom Mordenit-Typ als Träger unfaßt, in welchem Katalysator das Molverhältnis X:Y größer als $n^{-1}$ ist.

**2.** Verfahren nach Anspruch 1, worin das Metall X bzw. die Metalle X in den Katalysator durch Ionenaustausch eingebracht worden ist bzw. sind.

**3.** Verfahren nach Anspruch 1 oder 2, worin wenigstens eines der Metalle X zweiwertig ist.

**4.** Verfahren nach einem der vorstehenden Ansprüche, worin X Nickel umfaßt.

**5.** Verfahren nach einem der vorstehenden Ansprüche, worin Y wenigstens ein Metall, ausgewählt aus der aus Aluminium, Eisen, Gallium, Rhodium, Chrom und Scandium umfassenden Gruppe, umfaßt.

**6.** Verfahren nach einem der vorstehenden Ansprüche, worin das Molverhältnis Silizium:Y in dem Katalysator im Bereich von 5:1 bis 100:1 und vorzugsweise im Bereich von 7:1 bis 30:1 liegt.

**7.** Verfahren nach einem der vorstehenden Ansprüche, worin das Molverhältnis X:Y von 0,6 bis 1,5 und vorzugsweise von 0,8 bis 1,2 beträgt.

**8.** Verfahren nach einem der vorstehenden Ansprüche, worin der Katalysator aus einem Ammoniummordenit umfassenden Träger bereitet worden ist.

5

**9.** Verfahren nach einem der vorstehenden Ansprüche, worin das Einsatzmaterial alpha-Monoolefine umfaßt.

**10.** Verfahren nach einem der vorstehenden Ansprüche, das bei einer Temperatur von 150 bis 330°C, einem Druck von 1 bis 100 bar absolut und einer Raumgeschwindigkeit von 0,1 bis 10 kg Einsatzmaterial/Katalysator.Stunde ausgeführt wird.

**11.** Katalytisch wirksame Zusammensetzung, welche wenigstens ein n-wertiges Metall (X), ausgewählt aus der aus Metallen der Gruppen 1b, 2a, 2b, 4b, 5b, 6b, 7b und 8 des Periodensystems der Elemente bestehenden Gruppe, auf einem Silikat Von dreiwertigem Metall (Y) vom Mordenit-Typ als Träger umfaßt, in welcher Zusammensetzung das Molverhältnis X:Y größer als $n^{-1}$ ist.